# EUROPEAN PATENT SPECIFICATION

(11) **EP 4 201 310 B1**
(45) Date of publication and mention of the grant of the patent: **12.11.2025**
(21) Application number: 22211513.1
(22) Date of filing: 05.12.2022
(51) Int. Cl.: A61B 5/00, A61B 5/024, A61B 5/1455, A61B 5/18

(54) **SYSTEMS FOR MONITORING PILOT BIOMETRICS**
SYSTEME ZUR ÜBERWACHUNG DER BIOMETRIE VON PILOTEN
SYSTÈMES DE SURVEILLANCE DE DONNÉES BIOMÉTRIQUES DE PILOTE

(30) Priority: 21.12.2021 IN 202111059680; 07.04.2022 US 202217715494
(43) Date of publication of application: 28.06.2023
(73) Proprietor: Rockwell Collins, Inc., Cedar Rapids, IA 52498 (US)
(72) Inventor: BARDWAJ, Anish, 560066 Bengaluru, Karnataka (IN); CHAUHAN, Ruchita Singh, 560066 Bengaluru, Karnataka (IN); DASARI, Sneha Divya, 560066 Bengaluru, Karnataka (IN); KARINKA, Rashmitha Alva, 560066 Bengaluru, Karnataka (IN); VALIYAPARAMBIL, Praveen, 560066 Bengaluru, Karnataka (IN)
(74) Representative: Dehns

(56) References cited:
- WO-A1-2021/105314
- CN-U- 214 929 576
- US-A1- 2015 314 681
- US-A1- 2016 001 781
- US-A1- 2016 345 907
- US-A1- 2021 034 053

## Description

### TECHINCAL FIELD AND BACKGROUND

The present disclosure relates generally to safety improvements for aircraft, and more particularly, to systems and methods for monitoring pilot biometrics via a biometric device incorporated into a pilot seat belt assembly, the biometric device configured to communicate obtained biometrics to an aircraft interface device, which in turn is communicatively coupled to at least one of an aircraft communication system and an air traffic control system.

The Federal Aviation Administration (FAA) and like international agencies determine safety requirements for aircraft. Regarding pilots, the FAA mandates the required number of pilots present in the cockpit at all times during flight. While small aircraft can operate with a single pilot, the FAA mandates two-pilot operations for larger aircraft, and at least three-pilot operations for long-haul flights. Each pilot in the cockpit must be qualified and must undergo routine physical health examinations. While one pilot can typically fly the aircraft considering modern avionics, the at least one additional pilot provides redundancy of operations and typically shares the workload of the flying pilot by monitoring the instrumentation, communicating with air traffic control, etc. The monitoring pilot, commonly referred to as the "copilot," is present in the cockpit in the event of an emergency of the aircraft or a medical emergency of the flying pilot.

Medical emergencies can occur suddenly and without warning. In a single-pilot operation, a pilot medical emergency can be catastrophic. In a two-pilot operation, one pilot can assume control of the aircraft while the flight crew is available to assist the pilot suffering the medical emergency. To assist, cockpits are typically equipped with medical devices such as external defibrillators (AEDs) with EKG monitors, oxygen, resuscitative equipment, and various first aid kits, some of which include basic cardiac drugs. In the event of a medical emergency, procedure requires that the flying pilot, who may have assumed control of the aircraft, and/or flight crew communicate with air traffic control (ATC) and/or a physician-directed medical communications center to determine the course of action to be taken, which may include both medical instructions and flight instructions, for instance continuing to the destination, immediate landing, diversion, etc.

Existing procedures and systems for handling pilot medical emergencies aboard aircraft do not anticipate pilot distress and require communication from the at least one additional pilot and/or flight crew to return a response from the ground. As such, the existing procedures and systems are initiated only when a medical emergency has reached a critical level, and therefore do not provide adequate time to assess the extent of the emergency and best course of action to be taken for the both the pilot and aircraft. WO 2021/105314 A1 describes a method of determining fused sensor measurement and vehicle safety system using the fused sensor measurement. US 2021/0034053 A1 describes a pilot health and alertness monitoring, communication and safeguarding.

Therefore, what is needed are systems and methods for monitoring pilot biometrics continuously to anticipate the need for medical care and automatically alert the ground, the additional pilot(s) and flight crew to the possible medical emergency before the emergency elevates to a critical level. Such systems and methods would provide adequate and necessary time to develop a comprehensive plan for both the aircraft and the distressed pilot, potentially preventing loss of life, among other advantages.

### SUMMARY OF THE INVENTIVE ASPECTS

To achieve the foregoing and other advantages, the inventive aspects disclosed herein are broadly directed to systems and methods for monitoring pilot biometrics continuously to provide early notice of an escalating medical emergency so that appropriate steps can be taken regarding control of the aircraft and attention to the distressed pilot.

In a first aspect, the present disclosure provides a system for monitoring pilot biometrics continuously, as defined by claim 1.

In some embodiments, the at least one biometric device is embedded in webbing of the at least one over-shoulder strap.

In some embodiments, the at least one biometric device is carried in a sleeve slidably coupled to the at least one over-shoulder strap.

In some embodiments, the measured biometrics include at least one of heart rate, blood pressure and oxygen saturation.

In some embodiments, the predetermined condition is based on the measured biometrics exceeding a threshold biometrics value stored in a data storage of the aircraft interface device.

In some embodiments, the predetermined condition is based on trend activity of the measured biometrics.

In some embodiments, the at least one biometric device is communicatively coupled to the aircraft interface device via an interface module.

In some embodiments, the aircraft interface device is further configured to issue to the aircraft communication system, when the measured biometrics approaches the predetermined condition, a protocol stored in a data storage of the aircraft interface device indicating a first action to be taken by at least one of a pilot and the flight crew, and issue to the aircraft communication system, when the measured biometrics meets the predetermined condition, a protocol stored in the data storage of the aircraft interface device indicating a second action to be taken by at least one of the pilot and the flight crew.

### BRIEF DESCRIPTION OF THE DRAWINGS

Implementations of the concepts disclosed herein may be better understood when consideration is given to the following detailed description thereof. Such description makes reference to the included drawings, which are not necessarily to scale, and in which some features may be exaggerated, and some features may be omitted or may be represented schematically in the interest of clarity. Like reference numbers in the drawings may represent and refer to the same or similar element, feature, or function. In the drawings:
FIG. 1 is a schematic diagram showing a system for monitoring pilot biometrics according to an embodiment of the present disclosure;
FIG. 2 is a schematic diagram showing a seat belt assembly including an embedded biometrics device for measuring pilot biometrics continuously according to an embodiment of the present disclosure;
FIG. 3 is a schematic diagram showing a seat belt assembly including a sliding sleeve containing a biometrics device for measuring pilot biometrics continuously according to an embodiment of the present disclosure;
FIG. 4 is a schematic diagram showing a PPG sensor system according to an embodiment of the present disclosure;
FIG. 5 is a flowchart showing a method for monitoring and communicating measured biometrics according to an embodiment of the present disclosure; and
FIG. 6 is a system diagram showing communications from the at least one biometric device to the aircraft communication system and air traffic control.

### DETAILED DESCRIPTION

Unless expressly stated to the contrary, "or" refers to an inclusive or and not to an exclusive or. For example, a condition A or B is satisfied by anyone of the following: A is true (or present) and B is false (or not present), A is false (or not present) and B is true (or present), and both A and B are true (or present).

In addition, use of the "a" or "an" are employed to describe elements and components of embodiments of the instant inventive concepts. This is done merely for convenience and to give a general sense of the inventive concepts, and "a" and "an" are intended to include one or at least one and the singular also includes the plural unless it is obvious that it is meant otherwise.

Finally, as used herein any reference to "one embodiment," or "some embodiments" means that a particular element, feature, structure, or characteristic described in connection with the embodiment is included in at least one embodiment of the inventive concepts disclosed herein. The appearances of the phrase "in some embodiments" in various places in the specification are not necessarily all referring to the same embodiment, and embodiments of the inventive concepts disclosed may include one or more of the features expressly described or inherently present herein, or any combination of sub-combination of two or more such features, along with any other features which may not necessarily be expressly described or inherently present in the instant disclosure.

Broadly speaking, embodiments of the concepts disclosed herein are directed to systems and methods for monitoring, measuring and communicating pilot biometrics to an aircraft interface device communicatively coupled to at least one of air traffic control system and an aircraft communication system, such that air traffic control, additional pilots and flight crew can be kept apprised of an escalating medical emergency situation before the situation rises to a critical level.

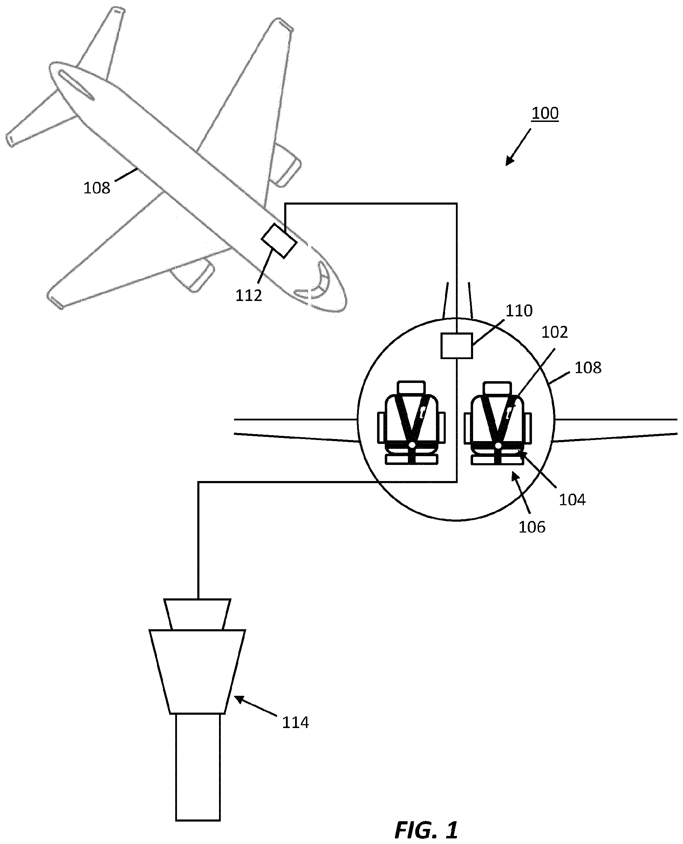

Referring to FIG. 1, the biometrics monitoring system 100 includes at least one biometrics device 102 located in a seat belt assembly 104 of a pilot seat 106. As used herein, the term "pilot" can also mean a copilot, the term "pilot seat belt assembly" can also mean a copilot seat belt assembly, and the term "pilot seat" can also mean a copilot seat. Thus, the systems and methods according to the present disclosure can be implemented for pilot and/or copilot seats in the cockpit. The biometrics device 102 is configured to measure biometrics continuously. The biometrics device 102 includes sensors, at least one processing module, and a communications module such as a network interface module. As shown, the at least one biometrics device 102 is embedded within or carried by an over-shoulder strap of the seat belt assembly 104 such that the at least one biometrics device 102 is positioned proximate the chest of the pilot in use.

In embodiments, the at least one biometrics device 102 includes a photoplethysmogram (PPG) sensor system. Known to those skilled in the art, PPG is a method for measuring the amount of light absorbed or reflected by blood vessels in living tissue. Since the amount of optical absorption or reflection depends on the amount of blood that is present in the optical path, the PPG signal is responsive to changes in the volume of the blood, rather than the pressure of the blood vessels. In other words, PPG detects the change of blood volume by the photoelectric technique, whether transmissive or reflective, to record the volume of blood in the sensor coverage area to form a PPG signal. The pulsatile component of a PPG signal is related to changes in blood volume inside the arteries and is synchronous with the heartbeat, whereas the non-pulsating component is a function of the basic blood volume, respiration, the sympathetic nervous system, and thermoregulation. Given that the optical absorption of hemoglobin is a function of oxygenation and optical wavelength, the use of PPG at multiple wavelengths can also be used in pulse oximetry. PPG technology represents a convenient technology that can be applied to various aspects of cardiovascular monitoring, including the detection of blood oxygen saturation, heart rate, blood pressure, cardiac output and respiration, among others. PPG offers the added advantage that it can be measured continuously using the wearable optical electronic device.

In the aircraft 108, each pilot seat 106 is equipped with the seat belt assembly 104 including at least one of the biometric devices 102. In some embodiments, a single biometric device 102 may be incorporated into each over-shoulder strap for redundancy, or for each device measuring a different biometric continuously. Each pilot seat 106 may be identified in the system, for instance by a 'pilot' or 'copilot' seat designation, by pilot name, 'first' or 'second' seat designation, etc. The seat identification may be communicated with the communicated measured biometrics, alarm or warning. In some embodiments, the biometric devices 102 form a network of biometric devices.

The biometric devices 102 communicate with the aircraft interface device 110, such as via a wireless connection. The aircraft interface device 110 in turn communicates with at least one of an aircraft communications system 112, via a wired or wireless communication, and an air traffic control system 114, via a wireless communication. Each biometric device 102 is configured to measure biometrics continuously and convey data associated with the measured biometrics to the aircraft interface device 110, for example via a network interface module of the biometrics device 102. The aircraft interface device 110 receives the information about the measured biometrics. Communicated and received information can include at least one of real-time biometrics data, warnings, alarms, etc. The aircraft interface device 110 may include a circuit having a processor and software for processing the received biometrics data. In embodiments, the biometric devices 102 or the aircraft interface device 110 generates waveforms from signals from the sensors and analyzes the signals or trends to provide early warnings and alarms, separately in some embodiments, to systems communicating with the aircraft interface device 110.

The at least one biometrics device 102 and/or the aircraft interface device 110 includes biometric data stored in data storage of the device against which the measured biometrics are compared. Measured biometric data falling within a predetermined acceptable range of the stored biometric data is designated 'Normal' biometric data, whereas measured biometric data falling outside of the predetermined acceptable range is designated 'Abnormal' biometric data. Regarding heart rate, for example, a 'Normal' heart rate range may be 60-100 beats per minute (BPM), which is a normal resting heart rate for adults. Considering that range, any measured heart rate within that range would be considered 'Normal,' whereas any measured heart rate less than 60 BPM or more than 100 BPM would be considered 'Abnormal.' Any measured heart rate qualifying as 'Abnormal' would be communicated as such, triggering at least one of a warning or alarm to the aircraft interface device 110 as discussed further below. Regarding oxygen saturation, for example, a 'Normal' reading may be 90% or greater, or 95% or greater, whereas an 'Abnormal' reading may be less than 90%, or less than 95%, respectively. As stated above, the predetermined 'Normal' stored data may be based on National averages or may be customized based on each individual pilot from historical data, for instance collected via a link with a medical provider.

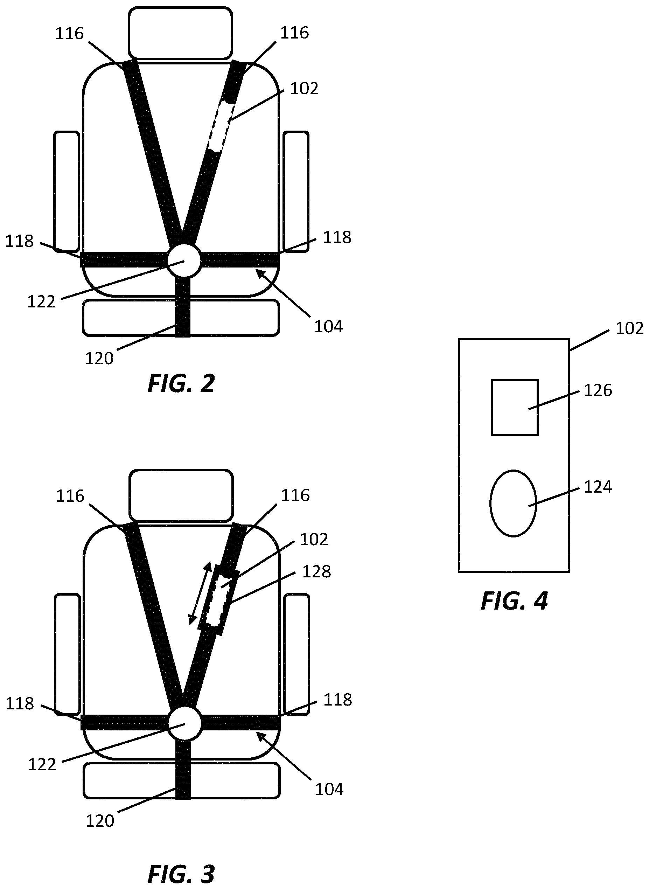

Referring to FIG. 2, in one embodiment the seat belt assembly 104 includes two over-shoulder straps 116, two waist straps 118, and a single crotch or "submarine" strap 120, each of which attaches to a single quick release 122. The collection of straps and release form a five-point harness. The biometrics device 102 is embedded in or attached to the webbing of one of the over-shoulder straps 116 such that the device 102, is positioned proximate the chest. Referring to FIG. 4, each biometrics device 102 includes a sensor 124 configured to emit light, and a detector 126 configured to detect reflected light, i.e., light reflected by blood vessels in living tissue. The biometrics device 102 further includes a network interface module for communicating the measured biometrics to the aircraft interface device. In some embodiments, the biometrics device 102 analyze the signals. In use, the over-shoulder strap 116 is positioned such that the sensor 124 and detector 126 are positioned facing the chest. The over-shoulder strap may include indicia designating a correct facing orientation and an incorrect facing orientation. In some embodiments, the biometric device communicates with at least one of a seat occupancy system and a seat belt attachment system such that, when a seat occupant is detected and the over-shoulder strap is inserted in the quick release, the biometrics system begins looking for the measured biometric. In the event the biometrics system times out without detecting the measurable biometric, the system may inform the aircraft interface device to a fault condition, which may be a faulty system or incorrect placement/orientation of the biometrics device 102.

Referring to FIG. 3, in another embodiment the seat belt assembly 104 as shown again includes two over-shoulder straps 116, two waist straps 118, and a single crotch or "submarine" strap 120, each of which attaches to a single quick release 122. The collection of straps and release again forms a five-point harness. In other embodiments the harness can be a four-point harness without a crotch strap, a three-point harness lacking waist straps, or other harness configurations. In the embodiment shown, the biometrics device 102 is incorporated into a sleeve 128 slidable along the length of its respective over-shoulder strap 116. In this configuration, the sleeve 128 is positionable along the length of the over-shoulder strap by sliding the sleeve to a comfortable worn position and/or for better biometrics readings. Referring again to FIG. 4, each biometrics device 102 includes a sensor 124, a detector 126, and a network interface module.

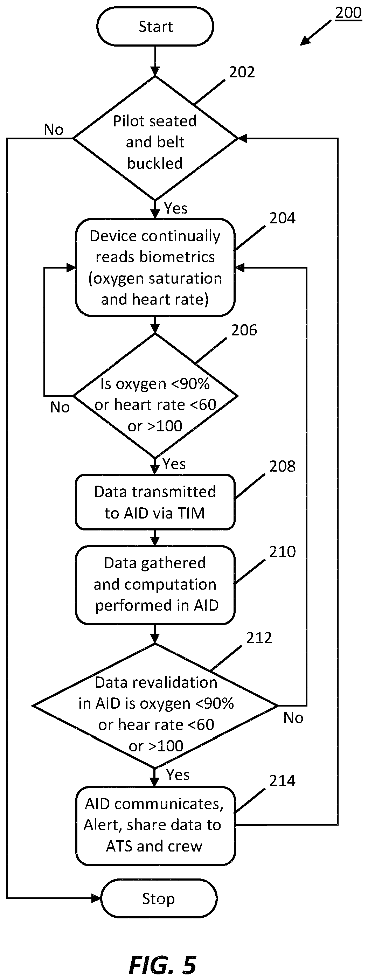

Referring to FIG. 5, a method for measuring pilot biometrics is shown generally at 200. As described above, the biometrics system 100 interfaces with a seat occupancy system with seat belt buckling detection. In a first Step 202, the system 100 determines if the pilot seat is occupied and/or the seat belt assembly, at least the over-shoulder strap carrying the biometrics device, is buckled. Upon confirmation that the pilot seat is occupied and the seat belt buckled, in a Step 204, the system 100 begins continuously measuring biometrics, for example heart rate and oxygen saturation. In some embodiments, the biometrics are measured continuously and reported in real-time to the aircraft interface device regardless of whether the measured biometrics are 'Normal' or 'Abnormal.' In a Step 206, the measured biometrics are compared against the stored ranges and values determined 'Normal.' When the measured biometrics are within the 'Normal' range, no biometric data may be reported to the aircraft interface device and the biometrics device continues to monitor the health parameters.

Continuing with the method 200, in a Step 208, when the measured biometrics fall outside of a 'Normal' range and thus are determined 'Abnormal' (e.g., oxygen saturation less than 90%, heart rate less than 60 BPM, heart rate greater than 100 BPM, etc.), the biometrics device reports the 'Abnormal' biometrics data to the aircraft interface device (AID), such as via the network interface module, where the data is gathered and in some cases computations performed in a Step 210. 'Abnormal' biometrics data reporting may be in the form of the actual numerical data, a warning or an alarm, or a combination thereof. In some embodiments, a warning communication may be triggered to issue when the biometric data approaches a limit of a 'Normal' range. For example, a measured heart rate of 65 BPM or 95 BPM may cause the system to issue a warning to the aircraft interface device that a threshold value is approaching. Warning issuances may be based on trends, rate increases over predetermined time periods, etc. An alarm may be issued when the measured biometrics are 'Abnormal' and the normal range exceeded. For example, an oxygen saturation less than 90%. In some embodiments, the first time the device reads an oxygen level drop the device may send an alert message to the aircraft communication system and the air traffic control system, and when the device detects an emergency reading continuously the device may send a warning message to the systems.

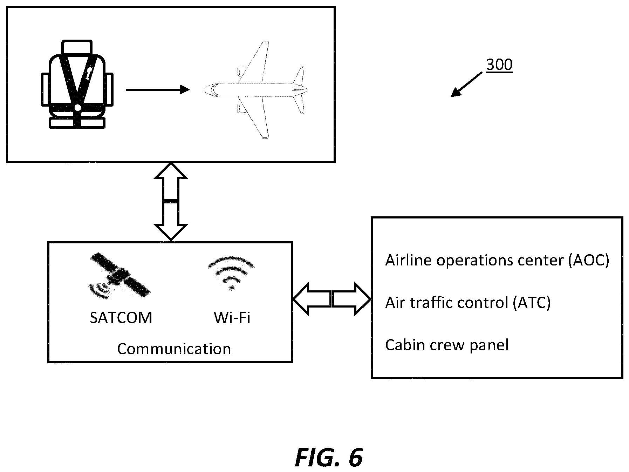

Continuing with the method 200, in a Step 212 the data may be validated to determine correctness of the measurement. Once validated, in a Step 214 the aircraft interface device communicates the 'Abnormal' biometric, in the form of numerical data, warning, alarm, etc., to at least one of the aircraft communication system and the air traffic control system ATS), for example, via the communications links of the system 300 shown in FIG. 6. In some embodiments, the aircraft communication system may be in communication with the additional pilots and/or flight crew. In a particular embodiment, the aircraft communication system includes a display for indicating to the crew the pilot under distress and the specific physical distress, optionally with medical instructions, such that the crew can react quickly to attend to the medical emergency. The display may be a flight crew control panel or other cabin display which provides other cabin information. Additional communications to the crew may include, but are not limited to, other visual and audio communications. Warnings and alarms may continue until acknowledged by the flight crew.

The same biometric data, warning and alarm information may be communicated to the air traffic control system. In response, the air traffic controller or receiving party can acknowledge the communication, contact the aircraft, and in some cases communicate with a medical expert who then conveys medical expertise to the flight crew via the air traffic controller or directly. For example, assume a cardiac arrest event of the flying pilot is measured by the biometrics device and is communicated to the aircraft interface device. The aircraft interface device in turn communicates the event to the aircraft communication system to notify the copilot and the crew, and the air traffic control system to notify the ground. The copilot receiving the communication can then react to assume control of the aircraft and the crew can react to lend medical assistance to the pilot. At the same time, air traffic control is made aware of the event and can help implement a flight plan for the aircraft, such as continuation, immediate landing, diversion, etc. The biometrics measuring may continue during the event such that, in the event the measured biometric returns to 'Normal,' the warning and alarm may be cancelled.

While the foregoing description provides embodiments of the invention by way of example only, it is envisioned that other embodiments may perform similar functions and/or achieve similar results.

## Claims

1. A system for monitoring pilot biometrics, comprising:
a seat belt assembly (104) comprising at least one over-shoulder strap (116);
at least one biometric device (102) carried by the at least one over-shoulder strap, the at least one biometric device including a photoplethysmogram, PPG, sensor system configured to measure biometrics continuously; and
an aircraft interface device (110) communicatively coupled to the at least one biometric device;
wherein;
the at least one biometric device is configured to obtain and communicate the measured biometrics to the aircraft interface device; and
the aircraft interface device is configured to communicate with an aircraft communication system and an air traffic control system, and when the measured biometrics meet a predetermined condition, issue an alert to the aircraft communication system and the air traffic control system informing that the predetermined condition has been met; wherein
the aircraft interface device is configured to, when the measured biometrics approaches the predetermined condition, issue a warning to the aircraft communication system and the air traffic control system; and
the aircraft interface device is configured to communicate with a flight crew display panel and cause the flight crew display panel to display the warning when the measured biometrics approaches the predetermined condition, and cause the flight crew display panel to display the alarm when the measured biometrics meets the predetermined condition

2. The system according to claim 1, wherein the at least one biometric device is embedded in webbing of the at least one over-shoulder strap.

3. The system according to claim 1, wherein the at least one biometric device is carried in a sleeve slidably coupled to the at least one over-shoulder strap.

4. The system according to any preceding claim, wherein the measured biometrics include at least one of heart rate, blood pressure and oxygen saturation.

5. The system according to any preceding claim, wherein the predetermined condition is based on the measured biometrics exceeding a threshold biometrics value stored in a data storage of the aircraft interface device.

6. The system according to any preceding claim, wherein the predetermined condition is based on trend activity of the measured biometrics.

7. The system according to any preceding claim, wherein the at least one biometric device includes a network interface module.

8. The system according to any preceding claim, wherein the aircraft interface device is further configured to:
issue to the aircraft communication system, when the measured biometrics approaches the predetermined condition, a protocol stored in a data storage of the aircraft interface device indicating a first action to be taken by at least one of a pilot and the flight crew, and
issue to the aircraft communication system, when the measured biometrics meets the predetermined condition, a protocol stored in the data storage of the aircraft interface device indicating a second action to be taken by at least one of the pilot and the flight crew.

## Patentansprüche

1. System zum Überwachen der Biometrie von Piloten, umfassend:
eine Sicherheitsgurtanordnung (104), umfassend mindestens einen Überschultergurt (116);
mindestens eine biometrische Vorrichtung (102), die durch den mindestens einen Überschultergurt getragen wird, wobei die mindestens eine biometrische Vorrichtung ein Photoplethysmogramm-Sensorsystem (photoplethysmogram, PPG) beinhaltet, das dazu konfiguriert ist, Biometrie kontinuierlich zu messen; und
eine Flugzeugschnittstellenvorrichtung (110), die kommunikativ mit der mindestens einen biometrischen Vorrichtung gekoppelt ist;
wobei;
die mindestens eine biometrische Vorrichtung dazu konfiguriert ist, die gemessene Biometrie zu erlangen und an die Flugzeugschnittstellenvorrichtung zu kommunizieren; und
die Flugzeugschnittstellenvorrichtung dazu konfiguriert ist, mit einem Flugzeugkommunikationssystem und einem Flugverkehrssteuerungssystem zu kommunizieren und, wenn die gemessene Biometrie eine vorbestimmte Bedingung erfüllt, eine Warnung an das Flugzeugkommunikationssystem und das Flugverkehrssteuerungssystem auszugeben, die darüber informiert, dass die vorbestimmte Bedingung erfüllt wurde;
wobei
die Flugzeugschnittstellenvorrichtung dazu konfiguriert ist, wenn sich die gemessene Biometrie dem vorbestimmten Zustand nähert, eine Warnung an das Flugzeugkommunikationssystem und das Flugsicherungssystem auszugeben; und
die Flugzeugschnittstellenvorrichtung dazu konfiguriert ist, mit einer Flugbesatzungs-Anzeigetafel zu kommunizieren und zu veranlassen, dass die Flugbesatzungs-Anzeigetafel die Warnung anzeigt, wenn sich die gemessene Biometrie dem vorgegebenen Zustand nähert, und zu veranlassen, dass die Flugbesatzungs-Anzeigetafel die Warnung anzeigt, wenn die gemessene Biometrie den vorbestimmten Zustand erfüllt.

2. System nach Anspruch 1, wobei die mindestens eine biometrische Vorrichtung in das Gurtband des mindestens einen Überschultergurts eingebettet ist.

3. System nach Anspruch 1, wobei die mindestens eine biometrische Vorrichtung in einer Hülle getragen wird, die verschiebbar mit dem mindestens einen Überschultergurt gekoppelt ist.

4. System nach einem der vorhergehenden Ansprüche, wobei die gemessene Biometrie mindestens eines von Herzfrequenz, Blutdruck und Sauerstoffsättigung beinhaltet.

5. System nach einem der vorhergehenden Ansprüche, wobei die vorbestimmte Bedingung darauf basiert, dass die gemessene Biometrie einen Biometrie-Schwellenwert überschreitet, der in einem Datenspeicher der Flugzeugschnittstellenvorrichtung gespeichert ist.

6. System nach einem der vorhergehenden Ansprüche, wobei der vorbestimmte Zustand auf der Trendaktivität der gemessenen Biometrie basiert.

7. System nach einem der vorhergehenden Ansprüche, wobei die mindestens eine biometrische Vorrichtung ein Netzwerkschnittstellenmodul beinhaltet.

8. System nach einem der vorhergehenden Ansprüche, wobei die Flugzeugschnittstellenvorrichtung ferner zu Folgendem konfiguriert ist:
Ausgeben eines in einem Datenspeicher der Flugzeugschnittstellenvorrichtung gespeicherten Protokolls an das Flugzeugkommunikationssystem, wenn sich die gemessene Biometrie der vorbestimmten Bedingung nähert, das eine erste Aktion anzeigt, die durch mindestens einen Piloten oder die Flugbesatzung durchzuführen ist; und
Ausgeben eines in dem Datenspeicher der Flugzeugschnittstellenvorrichtung gespeicherten Protokolls an das Flugzeugkommunikationssystem, wenn die gemessene Biometrie die vorbestimmten Bedingung erfüllt, das eine zweite Aktion anzeigt, die durch mindestens den Piloten oder die Flugbesatzung durchzuführen ist.

## Revendications

1. Système de surveillance de données biométriques de pilote, comprenant :
un ensemble de ceinture de sécurité (104) comprenant au moins une bandoulière (116) ;
au moins un dispositif de données biométriques (102) transporté par l'au moins une bandoulière, l'au moins un dispositif de données biométriques comportant un système de capteur photopléthysmographique, PPG, configuré pour mesurer des données biométriques en continu ; et
un dispositif d'interface de l'aéronef (110) couplé de manière communicative à l'au moins un dispositif de données biométriques ;
dans lequel ;
l'au moins un dispositif de données biométriques est configuré pour obtenir et communiquer les données biométriques mesurées au dispositif d'interface de l'aéronef ; et
le dispositif d'interface de l'aéronef est configuré pour communiquer avec un système de communication de l'aéronef et un système de commande de trafic aérien, et lorsque les données biométriques mesurées satisfont une condition prédéterminée, émettre une alerte au système de communication de l'aéronef et au système de commande de trafic aérien informant que la condition prédéterminée a été satisfaite ; dans lequel
le dispositif d'interface de l'aéronef est configuré pour, lorsque les données biométriques mesurées approchent de la condition prédéterminée, émettre un avertissement au système de communication de l'aéronef et au système de commande de trafic aérien ; et
le dispositif d'interface de l'aéronef est configuré pour communiquer avec un panneau d'affichage de l'équipage de bord et amener le panneau d'affichage de l'équipage de bord à afficher l'avertissement lorsque les données biométriques mesurées approchent de la condition prédéterminée, et amener le panneau d'affichage de l'équipage de bord à afficher l'alarme lorsque les données biométriques mesurées satisfont la condition prédéterminée.

2. Système selon la revendication 1, dans lequel l'au moins un dispositif de données biométriques est intégré dans la sangle de l'au moins une bandoulière.

3. Système selon la revendication 1, dans lequel l'au moins un dispositif de données biométriques est transporté dans un manchon couplé de manière coulissante à l'au moins une bandoulière.

4. Système selon une quelconque revendication précédente, dans lequel les données biométriques mesurées comportent au moins l'une de la fréquence cardiaque, la pression artérielle et la saturation en oxygène.

5. Système selon une quelconque revendication précédente, dans lequel la condition prédéterminée est basée sur les données biométriques mesurées dépassant une valeur de données biométriques seuil stockée dans un stockage de données du dispositif d'interface de l'aéronef.

6. Système selon une quelconque revendication précédente, dans lequel la condition prédéterminée est basée sur l'activité de tendance des données biométriques mesurées.

7. Système selon une quelconque revendication précédente, dans lequel l'au moins un dispositif de données biométriques comporte un module d'interface réseau.

8. Système selon une quelconque revendication précédente, dans lequel le dispositif d'interface de l'aéronef est également configuré pour :
émettre au système de communication de l'aéronef, lorsque les données biométriques mesurées approchent de la condition prédéterminée, un protocole stocké dans un stockage de données du dispositif d'interface de l'aéronef indiquant une première action à entreprendre par au moins l'un d'un pilote et de l'équipage de bord ; et
émettre au système de communication de l'aéronef, lorsque les données biométriques mesurées approchent de la condition prédéterminée, un protocole stocké dans un stockage de données du dispositif d'interface de l'aéronef indiquant une première action à entreprendre par au moins l'un du pilote et de l'équipage de bord.
